# EUROPEAN PATENT SPECIFICATION

(11) **EP 1 820 792 B1**
(45) Date of publication and mention of the grant of the patent: **18.01.2012**
(21) Application number: 06003477.4
(22) Date of filing: 21.02.2006
(51) Int. Cl.: C07C 209/62, C07C 211/38, C07C 233/06, C07C 335/32

(54) **Process for the preparation of adamantanamines**
Verfahren zur Herstellung von Adamantanaminen
Procédé pour la préparation des adamantanamines

(43) Date of publication of application: 22.08.2007
(73) Proprietor: Hexal AG, 83607 Holzkirchen (DE)
(72) Inventor: Schickaneder, Christian, 01309, Dresden (DE)
(74) Representative: Leger, Hubert

(56) References cited:
- GB-A- 1 393 503
- JIRGENSONS, A. ET AL.: "A practical synthesis of tert-alkylamines via the Ritter reaction with chloroacetonitrile" SYNTHESIS, no. 12, 2000, pages 1709-1712, XP002375279 ISSN: 0039-7881
- PLAKHOTNIK, V.M. ET AL.: "Adamantane derivatives. IV. Synthesis and antiviral activity of some amides containing the adamantyl group" PHARM. CHEM. J. (ENGL. TRANSL.), vol. 16, no. 9, 1982, pages 660-664, XP009070954
- DATABASE WPI Week 198207 Derwent Publications Ltd., London, GB; AN 1982-13098E XP002394924 -& SU 805 609 A1 (PETROVA I G) 30 September 1981 (1981-09-30)
- DATABASE CHEMCATS [Online] CHEMICAL ABSTRACTS SERVICE, COLUMBUS, OHIO, US; XP002394922 retrieved from STN & "ChemDiv, Inc. Product Library" 25 April 2003 (2003-04-25), CHEMDIV, INC., SAN DIEGO, CA, 92121 USA
- GERZON, K. ET AL.: "The Adamantyl Group in Medicinal Agents. I. Hypoglycemic N-Arylsulfonyl-N'-adamantylureas" JOURNAL OF MEDICINAL CHEMISTRY, AMERICAN CHEMICAL SOCIETY. WASHINGTON, US, vol. 6, no. 6, 1963, pages 760-763, XP002353954 ISSN: 0022-2623

## Description

The invention relates to a process for preparing certain adamantanamines, to intermediates used in the process, and to processes for preparing such intermediates.

Adamantanamines are known for long as a class of valuable pharmaceuticals. For example, Memantine hydrochloride (1-amino-3,5-dimethyladamantane hydrochloride, formula IVa) 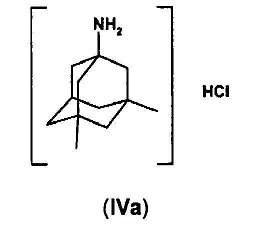 is an uncompetitive NMDA(*N*-methyl-D-aspartate) receptor antagonist, which is used as a new treatment for Alzheimer's disease. Memantine hydrochloride launched in the market as Axura® is the first AD therapeutic compound which acts via this mechanism of action. Memantine has been marketed for more than 20 years in Germany for the treatment of spasticity and dementia syndrome.

Amantadine hydrochloride (1-aminoadamantane, formula IVb) 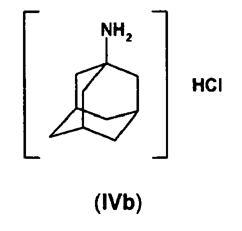 is an antiviral used to treat certain influenza infections (type A). The compound is also an antidyskinetic used to treat Parkinson's disease.

US 3,391,142 describes a process for preparing Memantine hydrochloride starting from 1-bromo-3,5-dimethyladamatane, which is reacted with acetonitrile in concencentrated sulphuric acid to yield the acetamido derivative, which is then cleaved under basic hydrolysis conditions.

According to WO 01/053234 adamantane is converted into N-(1-adamantyl) acetamide in a reaction medium containing dry acetonitrile, fluorine, and a lewis acid. This compound can be cleaved under acidic or basic conditions yielding amino adamantane.

To avoid a reaction with highly toxic HCN, several processes describe the reaction of dimethyladamantanes with urea or formamide (WO 05/023753, RU 2246482, DE 2318461, EP 0392059, CZ 288445).

There are a number of problems encountered with the conventional processes for preparing adamantanamines. The yields of at least some of the steps are low, the reaction time is relatively high, hazardous reaction conditions and/or solvents are required and/or the use of some solvents causes difficulties in purification of the final product.

Therefore there is a need for an improved process for preparing highly pure adamantanamines, in particular Memantine hydrochloride or Amantadine hydrochloride, which overcomes the above-mentioned problems and thereby provides a process which is economic and viable on a commercial scale.

The present invention therefore concerns a process for preparing a compound of formula (I) 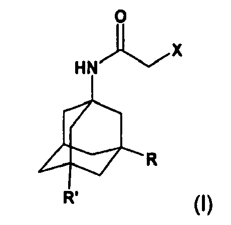 wherein X is chlorine and R and R' are each methyl, comprising reacting a compound of formula (III) 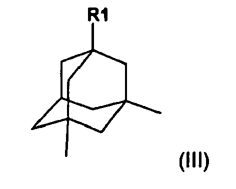 wherein R1 is bromine, with a haloacetonitrile X-CH₂-CN, wherein X is chlorine, in an acidic medium.

The acidic medium used for the reaction of the compound of formula (III) with the haloacetonitrile, in particular chloroacetonitrile, suitably comprises a strong mineral acid, in particular sulphuric acid. Further optional components of the acidic medium comprise one or more solvents, for example an organic acid, for example propionic acid or in particular acetic acid; and/or a polar aprotic solvent, for example N,N-dimethyl formamide (DMF); and a metal salt catalyst, for example iron(III) sulfate.

One embodiment of the invention comprises the reaction of 1-bromo-3,5-dimethyl adamantane (compound of formula (III), wherein R is bromo) with chloroacetonitrile in a solution comprising sulphuric acid, DMF, iron(III) sulfate, and optionally an organic acid, in particular acetic acid.

The reaction of the compound of formula (III) with the haloacetonitrile is advantageously carried out under mild conditions, for example at a temperature of from 0 to 70°C, preferably of from 0°C to room temperature and in particular of from 0 to 10°C. In general, the strong mineralic acid is added to the mixture of the reactants, solvent(s) and optional catalyst in a way that the temperature does not exceed the above-given limits. Following the addition of the mineral acid the reaction mixture, if necessary, may be kept at room temperature or at an elevated temperature, for example at a temperature from 50 to 100°C, preferably from 70 to 80°C, for a time suitable to complete the reaction, for example for up to 120 minutes and preferably for 30 to 90 minutes. Finally the reaction mixture is worked up in an usual manner, for example by hydrolyzing the reaction mixture with water at ambient conditions, washing, separating and drying the organic residue, thus obtaining the compound of formula (I), wherein R and R' are each methyl.

The compounds of formula (I), wherein R and R' are each hydrogen are known, for example, from A. Jirgensons et al., Synthesis 2000, 12, 1709 and may be obtained, for example, as described therein.

In a further aspect of the present invention the compound of formula (I) is reacted with thiourea according to the process of claim 9.

Compounds of formula (II) are subsequently treated in an acidic medium.

A preferred acidic medium is, for example, an aqueous acidic medium or a mixture of a C₁-C₄-alcohol and an acid or a mixture of a C₁-C₄-alcohol, water and an acid. A suitable acid in the conversion step of the compound of formula (II) is, for example, an organic C₁-C₄-carboxylic acid, for example acetic acid, propionic acid or butanoic acid, in particular acetic acid. Useful alcohols are as defined above, for example ethanol or isopropanol, In general, the compound of formula (II) is treated in the acidic medium under reflux for a time period sufficient to complete the conversion, which is, for example, a time period of up to 12 hours and preferably from 4 to 8 hours.

The reaction of the compounds of formula (I) with thiourea is advantageously carried out in an appropriate solvent at a temperature of, for example, from 60 to 120°C, preferably at reflux temperature of the reaction mixture, within a time period of, for example, from about 4 hours to about 10 hours. A suitable solvent is, for example a C₁-C₄-alcohol, for example methanol, ethanol or n- or isopropanol, in particular ethanol or isopropanol. The resulting compounds of formula (II) may be isolated in conventional manner, for example by removal of the solvent, washing, crystallizing and/or drying.

The step of isolating the compound of formula (II) may be omitted, and the compounds of formula (I) may be converted directly to the desired adamantanamine or a hydrohalogenid thereof by a treatment of the compound of formula (I) with thiourea, for example, under reflux in a reaction medium as described above, in particular in a reaction medium comprising an acid as described above, water and/or a C₁-C₄-alcohol.

The resulting adamantanamine in each case may be isolated from the reaction mixture in form of its free base, for example, by making the reaction solution alkaline and isolating and purifying the resulting product in known manner. For example, the reaction mixture is treated with an alkali hydroxide such as sodium hydroxide. Following the addition of a suitable organic solvent, a phase separation may be performed, and the organic layer then may be subjected to typical finishing steps such as concentration, crystallization, and/or drying steps.

The adamantanamine in form of its free base may be easily converted into the respective hydrohalogenide by a treatment with the respective hydrohalogenic acid in a medium comprising, for example a C₁-C₄-alcohol and optionally water. The adamantanamines, Memantine hydrochloride or Amantadine hydrochloride, are advantageously obtained by treating Memantine or Amantadine in a solution comprising a C₁-C₄-alcohol, in particular ethanol or isopropanol, and aqueous hydrochloric acid. Preferably, concentrated hydrochloric acid is added slowly to the solution of Memantine or Amantadine in a C₁-C₄-alcohol.

Instead of isolating Memantine or Amantadine in form of its free base, the above reaction mixture comprising the raw Memantine or Amantadine may be converted directly to the respective Memantine or Amantadine hydrohalogenide without isolation of the free base by adding the hydrohalogenic acid directly to the reaction mixture and isolating the Memantine or Amantadine hydrohalogenide, for example, as described above.

The examples further illustrate the present invention.

### Comparative Example 1

### Preparation of 2-Chloro-N-(3,5-dimethyl-adamantan-1-yl)-acetamide (Method 1)

A 0.50 l round-bottom three-necked flask, equipped with reflux condenser, oil ventile, mechanical stirrer, thermometer, and dropping funnel is charged with 1-hydroxy-dimethyl-adamantane (10.00 g, 55.47 mmol), chloroacetonitrile (8.38 g, 110.94 mmol), and acetic acid (17.74 ml, 307.48 mmol). The resulting suspension is cooled to 0 °C with vigorous stirring. To the cold reaction mixture is added sulphuric acid (96 %, 17.74 ml, 332.80 mmol), keeping the temperature below 10°C. After completion of addition the clear reaction mixture is allowed to reach room temperature. The resulting suspension is slowly treated with water (0 °C, 100 ml) and stirred for further 30 min at 0-5 °C. After separation, the colourless crystalline solid is washed in a stream of water (0 °C, 100 ml, 2 x) and dried under reduced pressure (40 °C, 72 h, 25 mbar) to give 13.25 g (51.80 mmol, 93.4 %) of 2-chloro-N-(3,5-dimethyl-adamantan-1-yl)-acetamide. **¹H NMR** (300 MHz, DMSO-d6, ppm): δ 7.66.(b, 1H, NH), 3.93 (s, 2H, RC*H*₂Cl), 2.08 (m, 1H, R₃C*H*), 1.75 (m, 2H, RC*H*₂R), 1.57 (m, 4 H, RC*H*₂R), 1.28 (m, 4H, RC*H*₂R), 1.11 (s, 2H, RC*H*₂R), 0.82 (s, 6H, RC*H*₃).**¹³C NMR** (75 MHz, DMSO-d6, ppm): δ 164.82 (CO), 52.73, 50.12, 46.69, 43.42, 42.17, 31.81, 29.43. **MS** (EI, m/z): 255 [M]⁺, 220 [M-Cl]⁺, 163 [M-C₂H₃ClNO]⁺. **IR** (KBr, cm⁻¹): 1661 s (V_{RCONHR}, Amide I), 1574 s (Amide II). **E.A.** (C H N, %): *calc*. for C₁₄H₂₂CINO · 0.1 HAc: C, 65.15; H, 8.00; N 5.15. *Found*: C 65.15, H 8.62, N 5.35. **mp.:** 106.8 °C.

### Comparative Example 2

### Preparation of 2-Chloro-N-(3,5-dimethyl-adamantan-1-yl)-acetamide (Method 2)

A 4.0 l round-bottom three-necked flask, equipped with reflux condenser, oil ventile, mechanical stirrer, thermometer, and dropping funnel is charged with 1-hydroxy-dimethyl-adamantane (47.50 g, 263.48 mmol), dimethylformamide (42.13 ml), chloroacetonitrile (39.81 g, 526.97 mmol), and acetic acid (84.27 ml, 1.46 mol). The resulting suspension is slowly treated with sulphuric acid (96 %, 84.27 ml, 1.58 mol) at room temperature with vigorous stirring upon which the reaction mixture reaches approximately 70°C. After completion of addition the clear reaction mixture is allowed to cool to room temperature. The resulting suspension is slowly treated with water (0 °C, 475 ml) and stirred for further 60 min at 0 - 5°C. After separation, the colourless crystalline solid is washed in a stream of water (0 °C, 475 ml, 2 x) and dried under reduced pressure (40 °C, 72 h, 25 mbar) to give 62.18 g (243.09 mmol, 92.26 %) of 2-chloro-N-(3,5-dimethyl-adamantan-1-yl)-acetamide.

### Comparative Example 3

### Preparation of 2-Chloro-N-(3,5-dimethyl-adamantan-1-yl)-acetamide (Method 3)

A 0.50 l round-bottom three-necked flask, equipped with reflux condenser, oil ventile, mechanical stirrer, thermometer, and dropping funnel is charged with 1-hydroxy-dimethyl-adamantane (10.00 g, 55.47 mmol), chloroacetonitrile (8.38 g, 110.94 mmol), dimethylformamide (8.87 ml) and acetic acid (17.74 ml, 307.48 mmol). The resulting suspension is slowly treated with sulphuric acid (96 %, 17.74 ml, 332.80 mmol) at room temperature with vigorous stirring upon which the reaction mixture reaches approximately 70 °C. After completion of addition the clear reaction mixture is slowly treated with water (15 °C, 100 ml) at 70 °C. Upon ceasing of the exothermic reaction the product crystallizes. The resulting suspension is cooled to 0 - 5 °C and stirred for further 60 min at this temperature. After filtration, the colourless crystalline solid is washed in a stream of water (20 °C, 50 ml, 4 x) and dried under reduced pressure (40 °C, 72 h, 25 mbar) to yield 13.71 g (53.59 mmol, 96.61 %) of 2-chloro-N-(3,5-dimethyl-adamantan-1-yl)-acetamide.

### Comparative Example 4

### Preparation of 2-Chloro-N-(3,5-dimethyl-adamantan-1-yl)-acetamide (Method 4)

A 0.50 l round-bottom three-necked flask, equipped with reflux condenser, oil ventile, mechanical stirrer, thermometer, and dropping funnel is charged with 1-hydroxy-dimethyl-adamantane (10.00 g, 55.47 mmol), chloroacetonitrile (8.38 g, 110.94 mmol), and acetic acid (17.74 ml, 307.48 mmol). The resulting suspension is slowly treated with sulphuric acid (96 %, 17.74 ml, 332.80 mmol) at room temperature with vigorous stirring upon which the reaction mixture reaches approximately 70 °C. After completion of addition the clear reaction mixture is slowly treated with water (15 °C, 100 ml) at 70 °C. Upon ceasing of the exothermic reaction the product crystallizes. The resulting suspension is cooled to 0 - 5 °C and stirred for further 60 min at this temperature. After filtration, the colourless crystalline solid is washed in a stream of water (20 °C, 50ml, 4 x) and dried under reduced pressure (40 °C, 72 h, 25 mbar) to yield 13.61 g (53.19 mmol, 95.89 %) of 2-chloro-N-(3,5-dimethyl-adamantan-1-yl)-acetamide.

### Example 5

### Preparation of 2- Chloro-N-(3,5-dimethyl-adamantan-1-yl) acetamide (Method 5)

A 0.25 l round-bottom three-necked flask, equipped with reflux condenser, oil ventile, mechanical stirrer, thermometer, and dropping funnel is charged with 1-bromo-3,5-dimethyladamantane compound (5.00 g, 20.56 mmol), iron(III)sulphate (84 mg, 0.21 mmol), dimethylformamide (6.00 ml), chloroacetonitrile (4.19 g, 55.50 mmol), and acetic acid (9.23 g, 153.73 mmol). The reaction mixture is cooled to 5 - 10 °C with stirring. Sulphuric acid (96 %, 8.87 ml, 166.4 mmol) is added drop wise, keeping the temperature at 5 - 10 °C. After completion of addition the resulting suspension is heated to 80 °C for about 90 min. The reaction mixture is allowed to cool to room temperature and hydrolyzed with water (50 ml). After cooling to 5 °C and stirring for further 30 min. the residue is separated and washed in a stream of water (150 ml) and dried under reduced pressure (40 °C, 72 h, 25 mbar) to yield 3.23 g (12.63 mmol, 61.4 %) of 2-chloro-N-(3,5-dimethyl-adamantan-1-yl)-acetamide as a colourless powder as described above.

### Example 6

### Preparation of 2- Chloro-N-(3,5-dimethyl-adamantan-1-yl) acetamide (Method 6)

A 0.25 1 round-bottom three-necked flask, equipped with reflux condenser, oil ventile, mechanical stirrer, thermometer, and dropping funnel is charged with 1-bromo-3,5-dimethyladamantane (5.00 g, 20.56 mmol), iron(III)sulphate (84 mg, 0.21 mmol) and dimethylformamide (6.00 ml). The reaction mixture is cooled to 5 - 10 °C with stirring. Sulphuric acid (96 %, 8.87 ml, 166.4 mmol) is added drop wise, keeping the temperature at 5 - 10 °C. After completion of addition the resulting suspension is treated with chloroacetonitrile (4.19 g, 55.50 mmol) at 5 °C and then heated to 70 - 80 °C for 30 min. The reaction mixture is allowed to cool to room temperature and hydrolyzed with water (50 ml). After cooling to 5 °C and stirring for further 30 min. the residue is separated and washed in a stream of water (150 ml) and dried under reduced pressure (40 °C, 72 h, 25 mbar) to yield 3.52 g (13.76 mmol, 66.9 %) of 2-chloro-N-(3,5-dimethyl-adamantan-1-yl)-acetamide as described above.

### Example 7

### Preparation of 2-Carbamimidosulfanyl-N-(3,5-dimethyl-adamantan-1-yl)acetamide

A 0.25 l round-bottom two-necked flask, equipped with reflux condenser, oil ventile and magnetic stir bar is charged with 2-chloro-N-(3,5-dimethyl-adamantan-1-yl)-acetamide (2.00 g, 7.82 mmol), ethanol (16 ml) and thiourea (718 mg, 9.43 mmol). The suspension is heated to reflux with stirring. After 10 min. the solvent is removed under reduced pressure resulting in a colourless oil. Upon addition of acetonitrile (76 ml) with stirring the residue crystallizes. The resulting suspension is cooled to 0 °C and stirring is continued for further 60 min. The colourless precipitate is separated, washed with acetonitrile (10 ml, 3 x) and dried under reduced pressure (40 °C, 60 mbar, 8 h) to yield 1.96 g (5.90 mmol, 75.5 %) of 2-carbamimidosulfanyl-N-(3,5-dimethyl-adamantan-1-yl)acetamid as a colourless powder. ¹H **NMR** (300 MHz, DMSO-d6, ppm): 9.52.(b, 1H, NH), 9.27.(b, 1H, NH), 3.90 (s, 2H, RC*H*₂S), 2.08 (m, 1H, R₃C*H*), 1.73 (m, 2H, RC*H*₂R), 1.57 (m, 4 H, RC*H*₂R), 1.28 (m, 4H, RC*H*₂R), 1.09 (s, 2H, RC*H*₂R), 0.80 (s, 6H, RC*H*₃). **¹³C NMR** (75 MHz, DMSO-d6, ppm): δ 170.13, 166.69 (CO), 53.21, 50.05, 46.49, 42.09, 34.07, 31.77, 29.92, 29.35. **MS** (ESI, m/z): 296.0 [M-Cl]⁺. **IR** (KBr, cm⁻¹): 1663 s (V_{RCONHR}, Amide I), 1553 s (Amide II). mp.: 212.3 °C (dec.). **E.A.** (C, H, N; %): *calc.* for C₁₅H₂₆CIN₃OS: C, 54.18; H, 7.90; N 12.66. *Found:* C 54.11, H 6.94, N 12.56.

### Example 8 Preparation of 1-Amino-3,5-dimethyladamantan (Memantine base)

A 0.50 l round-bottom three-necked flask, equipped with reflux condenser, oil ventile, mechanical stirrer, thermometer, and dropping funnel is charged with 2-chloro-N-(3,5-dimethyl-adamantan-1-yl)-acetamide (10.00 g, 39.10 mmol), ethanol (77.52 ml), acetic acid (15.5 ml), and thiourea (3.57 g, 46.92 mmol). The resulting suspension is heated to reflux (70 °C) and held at this temperature for 6 h with stirring. The turbid solution is made alkaline with sodium hydroxide (50 %, 15.5 ml) and treated with toluene at approximately 60 °C. A phase separation is performed, the aqueous layer is discarded. The organic layer is concentrated under vacuum, dissolved in *iso*-propanol (5 ml) and filtered. The residue is dried to give 5.22 g (29.12 mmol, 74.5 %) of 1-amino-3,5-dimethyladamantan as a liquid, which crystallizes upon storage. **¹H NMR** (300 MHz, DMSO-d6, ppm): δ 2.04 (m, 1H, R₃C*H*), 1.45 (b, 1.5 H, RN*H*₂), 1.33 (m, 2H, RC*H*₂R), 1.25-0.98 (m, 10 H, RC*H*₂R), 0.80 (s, 6H, RC*H*₃). **¹³C NMR** (75 MHz, DMSO-d6, ppm): δ 52.31, 50.39, 48.39, 44.43, 42.37, 32.16, 30.13, 30.00. **MS** (EI, m/z): 179 [M]⁺. **IR** (film, cm⁻¹): 3342, 3268 cm⁻¹ (m, ν_{NH}), 1593 (m, δ_{NH}).

### Example 9

### Preparation of 1-Amino-3,5-dimethyladamantan Hydrochloride (Memantine hydrochloride)

### (Method 1)

In a 0.05 l round-bottom flask equipped with magnetic stir bar, nitrogen inlet and oil ventile 1-amino-3,5-dimethyladamantan (2.69 g, 15.00 mmol) is dissolved in *iso*-propanol (2.7 ml). To the stirred clear solution aq. hydrochloric acid (31 %, 2 ml) is added drop wise at room temperature. The resulting suspension is cooled to 0 °C and stirred for further 60 min. The colourless crystalline solid is collected by filtration, washed with *iso*-propanol (0.5 ml, 3 x) and dried under reduced pressure (48 h, 30 °C, 50 mbar) to give 2.24 g (10.38 mmol, 69.2 %) of Memantine hydrochloride. **¹H NMR** (300 MHz, DMSO-d6, ppm): δ 8.24 (s, 3H, N*H*), 2.11 (m, 1H, R₃C*H*), 1.65 (m, 2H, RC*H*₂R), 1.46 (m, 4H, RC*H*₂R), 1.28 (s, 4H, RC*H*₂R), 1.10 (m, 2H, RC*H*₂R) 0.83 (s, 6H, RC*H*₃). **¹³C NMR** (75 MHz, DMSO-d6, ppm): δ 52.81, 49.96, 46.31, 41.96, 38.91, 32.37, 30.05, 29.54. **MS** (EI, m/z, free base): 179 [M]⁺. **IR** (KBr, cm⁻¹): 3435 (m, ν_{NH}). **mp**.: > 300 (subl.). **E.A.** (C, H, N; %): *calc*. for C₁₂H₂₂ClN·H₂O: C, 66.25; H, 10.28; N, 6.44. *Found*: C, 66.31 %; H, 9.40; N, 6.20. **GC** (rel. Ar.): 99.85 %.

### Example 10

### Preparation of 1-Amino-3,5-dimethyladamantan Hydrochloride (Memantine hydrochloride)

### (Method 2)

A 0.50 l round-bottom flask equipped with reflux condenser, oil ventile, mechanical stirrer, thermometer and dropping funnel is charged with 2-chloro-N-(3,5-dimethyl-adamantan-1-yl)-acetamide (10.00 g, 39.10 mmol), thiourea (3.571 g, 46.92 mmol), water (77.5 ml), and acetic acid (15.5 ml) is heated to reflux and held at this temperature for 6 h with stirring. The reaction mixture is allowed to cool to approximately 60 °C and is treated drop wise with aq. hydrochloric acid (31 %, 25 ml). The resulting suspension is cooled to room temperature. The precipitate is collected by filtration, washed with water (10 ml) and dried under reduced pressure (30 °C, 25 mbar, 12 h) to give 5.78 g (26.87 mmol, 68.7 %) of Memantine hydrochloride as a colourless crystalline solid as described in example 9.

### Example 11

### Preparation of 1-Amino-3,5-dimethyladamantan Hydrochloride (Memantine hydrochloride)

### (Method 3)

A 4.0 l round-bottom three-necked flask, equipped with reflux condenser, oil ventile, mechanical stirrer, thermometer, and dropping funnel is charged with 2-chloro-N-(3,5-dimethyl-adamantan-1-yl)-acetamide (106.7 g, 0.42 mol), thiourea (38.1 g, 0.50 mol), acetic acid (165.4 ml), and ethanol (827.1 ml). The reaction mixture is heated to reflux with stirring and held for 8 h at this temperature. After completion of the reaction the vessel contents are allowed to reach room temperature. The turbid solution is filtered and the clear filtrate is treated first with water (1070 ml), then with aq. sodium hydroxide (50 %, 165.4 ml) without external cooling in a period of 15 min. Temperature may reach up to 60 °C. Toluene (215 ml) is added and the mixture is vigorously stirred for 15 min. A phase separation is performed. The aqueous phase is extracted once with toluene (220 ml) at 45 - 55 °C. The phases are separated, organic layers are combined, aqueous layers discarded. To the combined organic phases sodium hydroxide is added (50%, 100 ml) and the two phase system is vigorously stirred at 45 - 55 °C for 15 min. The phases are separated, the aqueous layer is discarded. The organic layer is treated with water (100 ml), making sure the temperature is maintained between 45 - 55 °C. The phases are separated, the aqueous layer is discarded. The organic layer is treated with sodium chloride (aq., conc, 100 ml) with stirring, maintaining the temperature between 45 - 55 °C. A phase separation is performed, the aqueous layer is disposed. The organic phase is filtered and transferred to a 2.0 l round-bottom three-necked flask, equipped with reflux condenser, oil ventile, mechanical stirrer, thermometer, and dropping funnel. The vessel contents are cooled to 0 - 5 °C with stirring. Aq. hydrochloric acid (31 %, 53 ml) is added, keeping the temperature below 20 °C. After completion of addition, the resulting suspension is cooled to 0 - 5 °C and stirred for further 30 min. The colourless, microcrystalline solid is collected by filtration, washed with toluene (100 ml, 3 x) and dried under reduced pressure (40 °C, 30 mbar, 18.5 h) to give 71.25 g (0.33 mol, 71.16 %) of Memantine hydrochloride as crystalline solid as described in example 9.

### Comparative Example 12

### Synthesis of N-Adamantan-1-yl-2-chloro-acetamide

A 0.50 l round-bottom three-necked flask, equipped with reflux condenser, oil ventile, mechanical stirrer, thermometer, and dropping funnel is charged with 1-hydroxyadamantane (10.00 g, 65.69 mmol), chloroacetonitrile (9.92 g, 131.38 mmol), dimethylformamide (11 ml) and acetic acid (21 ml, 363.96 mmol). The resulting suspension is slowly treated with sulphuric acid (96 %, 21 ml) at room temperature with vigorous stirring upon which the reaction mixture reaches approximately 80 °C. After completion of addition the clear reaction mixture is slowly treated with water (15 °C, 118 ml) at 70 °C. Upon ceasing of the exothermic reaction the product crystallizes. The resulting suspension is cooled to 0 - 5 °C with stirring. After filtration, the colourless crystalline solid is washed in a stream of water and dried under reduced pressure (40 °C, 19 h, 25 mbar) to yield 14.90 g (65.43 mmol, 99.60 %) of *N-*Adamantan-1-yl-2-chloro-acetamide. **¹H NMR** (300 MHz, CDCl₃, ppm): 6.24 (b,1H, N*H*), 3.94 (s, 2H, RC*H*₂Cl), 2.10 (m, 3H, R₃C*H*), 2.03 (m, 6H, RC*H*₂R), 1.70 (m, 6H, RC*H*₂R). **¹³C NMR** (75 MHz, DMSO-d6, ppm): δ 164.61 (CO), 52.39, 42.89, 41.37, 36.22, 29.38. **MS** (EI, m/z): 227 [M]⁺. **IR** (KBr, cm⁻¹): 1662 s (ν_{RCONHR}, Amide I), 1569 s (Amide II). **mp**.: 123.4 °C.

### Comparative Example 13

### Prep. of N-Adamantan-1-yl-2-carbamimidoylsulfanyl-acetamide Hydrochloride

A 0.25 l round-bottom two-necked flask, equipped with reflux condenser, oil ventile and magnetic stir bar is charged with *N*-adamantan-1-yl-2-chloro-acetamide (2.00 g, 8.78 mmol), ethanol (16 ml) and thiourea (802 mg, 10.54 mmol). The suspension is heated to reflux with stirring. After 15 min. the solvent is removed under reduced pressure. Acetonitrile (76 ml) is added to the oily residue with stirring. The resulting suspension is stirred for further 30 min. The colourless precipitate is separated at room temperature, washed with acetonitrile (10 ml, 3 x) and dried under reduced pressure (40 °C, 60 mbar, 19 h) to yield 2.18 g (7.16 mmol, 81.6 %) of the title compound as colourless, microcrystalline powder. **¹H NMR** (300 MHz, DMSO-d6, ppm): 9.42 (b, 3H, NH), 8.32 (s, 1H, NH), 3.95 (s, 2H, RC*H*₂S), 2.07-1.81 (m, 9H, R₃C*H*, RC*H*₂R), 1.61 (s, 6H, RC*H*₂R).**¹³C NMR** (75 MHz, DMSO-d6, ppm): δ 170.18, 166.65 (CO), 51.67, 40.57, 35.84, 34.14, 28.70. **MS** (ESI, m/z): 298.0 [MH-Cl]⁺. **IR** (KBr, cm⁻¹): 1662 s (ν_{RCONHR}, Amide I), 1555 s (Amide II). **mp**.: 180 °C (dec.). **E.A.** (C, H, N; %): *calc*. for C₁₅H₂₆CIN₃OS· 0.1 H₂O: C, 51.08; H, 7.32; N 13.75 *Found:* C 51.07, H 7.22, N 13.65.

### Comparative Example 14

### Synthesis of Amantadine HCl

A 2.0 l round-bottom three-necked flask, equipped with reflux condenser, oil ventile, mechanical stirrer, thermometer, and dropping funnel is charged with *N*-adamantan-1-yl-2-chloro-acetamide (71.0 g, 0.31 mol), thiourea (28.5 g, 0.37 mol), acetic acid (123.6 ml), and ethanol (550.2 ml). The reaction mixture is heated to reflux with stirring and held for 8 h at this temperature. After completion of the reaction the vessel contents are allowed to reach room temperature. The suspension is filtered over cellites and the clear filtrate is treated first with water (710 ml), then with aq. sodium hydroxide (50 %, 124 ml) without external cooling in a period of 10 min. Temperature may reach up to 50 °C. Toluene (142 ml) is added and the mixture is vigorously stirred for 15 min. A phase separation is performed. The aqueous phase is extracted once with toluene (142 ml) at 45 - 55 °C. The phases are separated, organic layers are combined, aqueous layers discarded. To the combined organic phases sodium hydroxide is added (50%, 71 ml) and the two phase system is vigorously stirred at 45 - 55 °C for 15 min. The phases are separated, the aqueous layer is discarded. The organic layer is diluted with toluene (20 ml) and washed with sodium hydroxide (50%, 71 ml) at 50 - 60 °C, aqueous layers are discarded. The organic layer is treated with water (71 ml), making sure the temperature is maintained between 50 - 60 °C. The phases are separated, the aqueous layer is discarded. The organic layer is treated with sodium chloride (aq., sat., 71 ml) with stirring, maintaining the temperature between 45 - 55 °C. A phase separation is performed, the aqueous layer is disposed. The organic phase is filtered over cellite and transferred to a 2.0 l round-bottom three-necked flask, equipped with reflux condenser, oil ventile, mechanical stirrer, thermometer, and dropping funnel. Transfer lines are flushed with toluene (20 ml, 2 x). The vessel contents are cooled to 0 - 5 °C with stirring. Aq. hydrochloric acid (31 %, 53 ml) is added, keeping the temperature below 20 °C. After completion of addition, the resulting suspension is cooled to 0 - 5 °C and stirred for further 60 min. The colourless, microcrystalline solid is collected by filtration, washed with toluene and dried under reduced pressure (40 °C, 30 mbar, 18.5 h) to give 47.34 g (0.25 mol, 80.89 %) of Amantadine HCl as microcrystalline solid. **mp.** > 300 °C (subl.). **¹H NMR** (300 MHz, DMSO-d6, ppm): δ 7.24.(b, 3H, NH), 2.06 (m, 3H, R₃C*H*), 1.82 (s, 6H, RC*H*₂R), 1.60 (m, 6H, RC*H*₂R).

## Claims

1. A process for preparing a compound of formula (I) 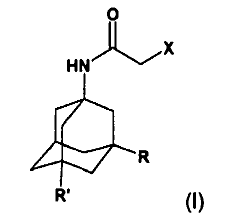 wherein X is chlorine and R and R' are each methyl, comprising reacting a compound of formula (III) 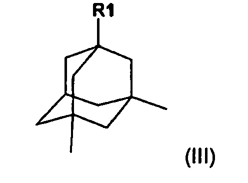 wherein R1 is bromine, with a haloacetonitrile X-CH₂-CN, wherein X is chlorine, in an acidic medium.

2. The process according to claim 1 wherein the acidic medium comprises a strong mineral acid.

3. The process according to claim 2 wherein the strong mineral acid is sulphuric acid.

4. The process according to any preceding claim wherein the acidic medium comprises a metal salt catalyst.

5. The process according to claim 4 wherein the metal salt catalyst is iron (III) sulphate.

6. The process according to any preceding claim wherein the acidic medium comprises one or more solvents selected from an organic acid and a polar aprotic solvent.

7. The process according to claim 6 wherein the organic acid is selected from propionic acid and acetic acid.

8. The process according to claim 6 wherein the polar aprotic solvent is N,N-dimethyl formamide.

9. The process according to any preceding claim wherein the compound of formula (I) is reacted with thiourea to form a compound of formula (II)

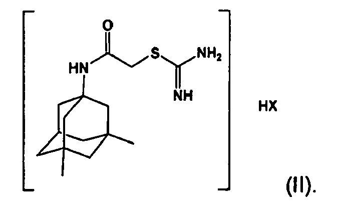

10. The process according to claim 9 wherein the compound of formula (II)is subjected to an acid treatment and the resulting adamantanamine of formula (IV) 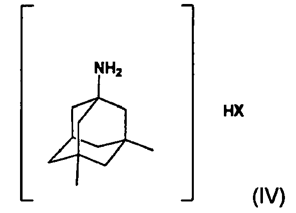 wherein X is chlorine, is isolated.

11. The process according to claim 10, wherein the acid treatment comprises treating the compound of formula (II) in a medium comprising a C₁-C₄-carboxylic acid and one or more solvents selected from the group consisting of water and a C₁-C₄-alcohol.

12. The process according to claim 11, wherein the C₁-C₄-carboxylic acid is acetic acid,

13. The process according to claims 10 to 12, wherein the adamantanamine is isolated as the free base by making the reaction solution alkaline.

14. The process according to claim 13, comprising, as an additional step, the conversion of the free adamantanamine base to an adamantanamine hydrohalogenide by a treatment with a hydrohalogenic acid, in particular with hydrochloric acid.

15. The process according to any preceding claim, wherein the compound of formula (I) is converted directly to the adamantanamine without isolation of the compound of formula (II) by reaction with thiourea in a medium comprising a C₁-C₄-carboxylic acid and one or more solvents selected from the group consisting of water and a C₁-C₄-alcohol.

16. The process according to claim 15, wherein the adamantanamine is converted directly to the adamantanamine hydrohalogenide without isolation of the free base by adding the hydrohalogenic acid to the reaction mixture comprising the raw adamantanamine.

## Patentansprüche

1. Ein Verfahren für die Herstellung einer Verbindung mit der Formel (I), 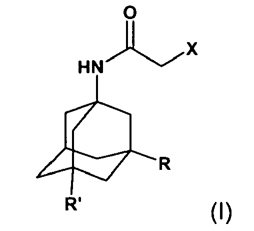 bei der X Chlor ist und R und R' jeweils Methyl sind, umfassend das Umsetzen einer Verbindung mit der Formel (III), 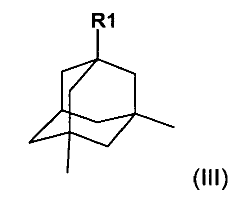 bei der R1 Brom ist, mit einem Haloacetonitril X-CH₂-CN, wobei X Chlor ist, in einem sauren Medium.

2. Das Verfahren nach Anspruch 1, wobei das saure Medium eine starke Mineralsäure umfasst.

3. Das Verfahren nach Anspruch 2, wobei es sich bei der starken Mineralsäure um Schwefelsäure handelt.

4. Das Verfahren nach einem der vorhergehenden Ansprüche, wobei das saure Medium einen Metallsalz-Katalysator umfasst.

5. Das Verfahren nach Anspruch 4, wobei es sich bei dem Metallsalz-Katalysator um Eisen(III)-Sulfat handelt.

6. Das Verfahren nach einem der vorhergehenden Ansprüche, wobei das saure Medium ein oder mehrere Lösungsmittel umfasst, die aus einer organischen Säure und einem polaren aprotischen Lösungsmittel ausgewählt werden.

7. Das Verfahren nach Anspruch 6, wobei die organische Säure aus Propionsäure und Essigsäure ausgewählt wird.

8. Das Verfahren nach Anspruch 6, wobei es sich bei dem polaren aprotischen Lösungsmittel um N,N-Dimethylformamid handelt.

9. Das Verfahren nach einem der vorhergehenden Ansprüche, wobei die Verbindung mit Formel (I) mit Thioharnstoff zur Reaktion gebracht wird, um die Verbindung mit Formel (II) zu erzeugen

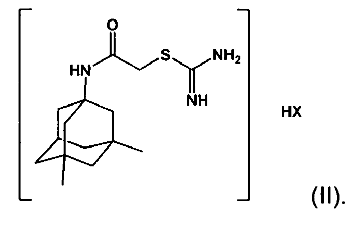

10. Das Verfahren nach Anspruch 9, wobei die Verbindung von Formel (II) mit Säure behandelt wird und das entstehende Adamantanamin mit Formel (IV), 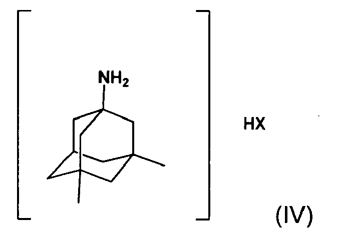 bei dem X Chlor ist, isoliert wird.

11. Das Verfahren nach Anspruch 10, wobei die Säurebehandlung die Behandlung der Verbindung mit Formel (II) in einem Medium umfasst, welches eine C₁-C₄-Carbonsäure und ein oder mehrere Lösungsmittel aus der Gruppe, die aus Wasser und einem C₁-C₄-Alkohol besteht, umfasst.

12. Das Verfahren nach Anspruch 11, wobei es sich bei der C₁-C₄-Carbonsäure um Essigsäure handelt.

13. Das Verfahren nach den Ansprüchen 10 bis 12, wobei das Adamantanamin als freie Base isoliert wird, indem die Reaktionslösung alkalisch gemacht wird.

14. Das Verfahren nach Anspruch 13, **dadurch gekennzeichnet, dass** es als zusätzlichen Schritt die Umwandlung der freien Adamantanamin-Base in ein Adamantanamin-Hydrohalogenid umfasst, durch die Behandlung mit einer Halogenwasserstoffsäure, insbesondere mit Chlorwasserstoffsäure.

15. Das Verfahren nach einem der vorhergehenden Ansprüche, wobei die Verbindung mit Formel (I) direkt in das Adamantanamin umgewandelt wird, ohne die Isolation der Verbindung mit Formel (II) durch die Reaktion mit Thioharnstoff in einem Medium, das C₁-C₄-Carbonsäure sowie ein oder mehrere Lösungsmittel umfasst, die aus einer Gruppe bestehend aus Wasser und einem C₁-C₄-Alkohol ausgewählt werden.

16. Das Verfahren nach Anspruch 15, wobei das Adamantanamin direkt in das Adamantanamin-Hydrohalogenid umgewandelt wird, ohne die Isolation der freien Base durch Hinzufügen der Halogenwasserstoffsäure zu der Reaktionsmischung, die das rohe Adamantanamin enthält.

## Revendications

1. Un procédé de préparation d'un composé de formule (I) 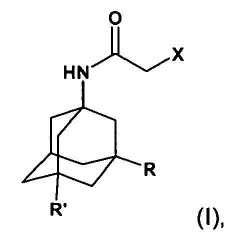 où X est du chlore, R et R' sont des groupes méthyle, comprenant la réaction d'un composé de formule (III) 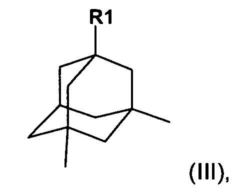 où R1 est du brome, avec un haloacétonitrile X-CH₂-CN, où X est du chlore, en milieu acide.

2. Le procédé selon la revendication 1, où le milieu acide comprend un acide minéral fort.

3. Le procédé selon la revendication 2, où l'acide minéral fort est de l'acide sulfurique.

4. Le procédé selon toute revendication précédente, où le milieu acide comprend un sel métallique en tant que catalyseur.

5. Le procédé selon la revendication 4, où le sel métallique catalyseur est du sulfate de fer (III).

6. Le procédé selon toute revendication précédente, où le milieu acide comprend un ou plusieurs solvants sélectionnés à partir d'un acide organique et d'un solvant aprotique polaire.

7. Le procédé selon la revendication 6, où l'acide organique est sélectionné à partir de l'acide propénoïque et de l'acide acétique.

8. Le procédé selon la revendication 6, où le solvant aprotique polaire est du N,N-diméthylformamide.

9. Le procédé selon toute revendication précédente, où le composé de formule (I) est réagi avec de la thio-urée pour former un composé de formule (II)

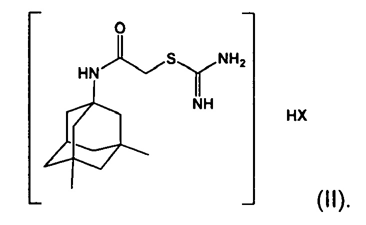

10. Le procédé selon la revendication 9, où le composé de formule (II) est soumis à un traitement acide, et l'adamantanamine en résultant, de formule (IV) 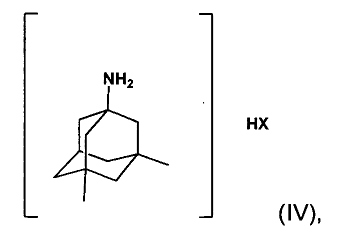 où X est du chlore, est isolée.

11. Le procédé selon la revendication 10, où le traitement acide comprend le traitement du composé de formule (II) dans un milieu comprenant un acide carboxylique C₁-C₄ et un ou plusieurs solvants sélectionnés dans un groupe constitué d'eau et d'un alcool C₁-C₄.

12. Le procédé selon la revendication 11, où l'acide carboxylique C₁-C₄ est de l'acide acétique.

13. Le procédé selon les revendications 10 et 12, où l'adamantanamine est isolée en tant que base libre, en rendant la solution de réaction alcaline.

14. Le procédé selon la revendication 13, comprenant, en guise d'étape supplémentaire, la conversion de la base libre d'adamantanamine en hydrohalogénure d'adamantanamine par traitement avec un acide hydrohalogénique, en particulier avec de l'acide hydrochlorique.

15. Le procédé selon toute revendication précédente, où le composé de formule (I) est converti directement en adamantanamine sans isolation du composé de formule (II) par réaction avec de la thio-urée dans un milieu comprenant un acide carboxylique C₁-C₄ et un ou plusieurs solvants sélectionnés dans un groupe constitué d'eau et d'un alcool C₁-C₄.

16. Le procédé selon la revendication 15, où l'adamantanamine est convertie directement en hydrohalogénure d'adamantanamine sans isolation de la base libre par ajout d'acide hydrohalogénique au mélange réactionnel comprenant l'adamantanamine pure.
